# EUROPEAN PATENT APPLICATION

(11) **EP 3 741 411 A1**
(43) Date of publication of application: **25.11.2020**
(21) Application number: 19176368.9
(22) Date of filing: 24.05.2019
(51) Int. Cl.: A61M 11/06, A24F 47/00, A61M 15/00, A61M 15/06

(54) **AEROSOL DELIVERY DEVICE**

(71) Applicant: NERUDIA LIMITED, Liverpool Merseyside L24 9HP (GB)
(72) Inventor: The designation of the inventor has not yet been filed
(74) Representative: Mewburn Ellis LLP

(57) **Abstract**

An aerosol delivery device comprising: an aerosol generator configured to produce an aerosol from an aerosol precursor during a delivery event, wherein the aerosol comprises a flavour component; and a switching device permitting switching of the aerosol generator between a normal mode and a boost mode, wherein the aerosol generator is configured to: in the normal mode, produce aerosol comprising a first mass of aerosol precursor per delivery event; and in the boost mode, produce aerosol comprising a second mass of aerosol precursor per delivery event, wherein the second mass is greater than the first mass.

## Description

### Field of the Invention

The present invention relates to an aerosol delivery device and particularly, although not exclusively, an aerosol delivery device operable in a normal mode and a boost mode.

### Background

A smoking-substitute device is an electronic device that permits the user to simulate the act of smoking by producing an aerosol mist or vapour that is drawn into the lungs through the mouth and then exhaled. The inhaled aerosol mist or vapour typically bears nicotine and/or other flavourings without the odour and health risks associated with traditional smoking and tobacco products. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

One approach for a smoking substitute device is the so-called "vaping" approach, in which a vaporisable liquid, typically referred to (and referred to herein) as "e-liquid", is heated by a heating device to produce an aerosol vapour which is inhaled by a user. The e-liquid typically includes a base liquid as well as nicotine and/or flavourings. The resulting vapour therefore also typically contains nicotine and/or flavourings. The base liquid may include propylene glycol and/or vegetable glycerine.

A typical vaping smoking substitute device includes a mouthpiece, a power source (typically a battery), a tank for containing e-liquid, as well as a heating device. In use, electrical energy is supplied from the power source to the heating device, which heats the e-liquid to produce an aerosol (or "vapour") which is inhaled by a user through the mouthpiece.

Vaping smoking substitute devices can be configured in a variety of ways. For example, there are "closed system" vaping smoking substitute devices, which typically have a sealed tank and heating element. The tank is pre-filled with e liquid and is not intended to be refilled by an end user. One subset of closed system vaping smoking substitute devices include a main body which includes the power source, wherein the main body is configured to be physically and electrically coupled to a consumable including the tank and the heating element. The consumable may also be referred to as a cartomizer. In this way, when the tank of a consumable has been emptied, that consumable is disposed of. The main body can be reused by connecting it to a new, replacement, consumable. Another subset of closed system vaping smoking substitute devices are completely disposable, and intended for one-use only.

There are also "open system" vaping smoking substitute devices which typically have a tank that is configured to be refilled by a user. In this way the device can be used multiple times.

An example vaping smoking substitute device is the myblu™ e-cigarette. The myblu™ e cigarette is a closed system device which includes a main body and a consumable. The main body and consumable are physically and electrically coupled together by pushing the consumable into the main body. The main body includes a rechargeable battery. The consumable includes a mouthpiece, a sealed tank which contains e-liquid, as well as a heating device, which for this device is a heating filament coiled around a portion of a wick. The wick is partially immersed in the e-liquid, and conveys e-liquid from the tank to the heating filament. The device is activated when a microprocessor on board the main body detects a user inhaling through the mouthpiece. When the device is activated, electrical energy is supplied from the power source to the heating device, which heats e-liquid from the tank to produce a vapour which is inhaled by a user through the mouthpiece.

For a smoking substitute device it is desirable to deliver nicotine into the user's lungs, where it can be absorbed into the bloodstream. As explained above, in the so-called "vaping" approach, "e-liquid" is heated by a heating device to produce an aerosol vapour which is inhaled by a user. Many e-cigarettes also deliver flavour to the user, to enhance the experience. Flavour compounds are contained in the e-liquid that is heated. Heating of the flavour compounds may be undesirable as the flavour compounds are inhaled into the user's lungs. Toxicology restrictions are placed on the amount of flavour that can be contained in the e-liquid. This can result in some e-liquid flavours delivering a weak and underwhelming taste sensation to consumers in the pursuit of safety.

In aerosol delivery devices, it is desirable to avoid large liquid droplets reaching a user's mouth.

The present invention has been devised in light of the above considerations.

### Summary of the Invention

At its most general, the present invention relates to an aerosol delivery device which is switchable between: a normal mode in which an aerosol comprising a first mass of aerosol precursor is produced; and a boost mode in which an aerosol comprising a second mass of aerosol precursor is produced, wherein the second mass is greater than the first mass.

According to the present invention, there is provided an aerosol delivery device comprising: an aerosol generator configured to produce an aerosol from an aerosol precursor during a delivery event, wherein the aerosol comprises a flavour component; and a switching device permitting switching of the aerosol generator between a normal mode and a boost mode, wherein the aerosol generator is configured to: in the normal mode, produce aerosol comprising a first mass of aerosol precursor per delivery event; and in the boost mode, produce aerosol comprising a second mass of aerosol precursor per delivery event, wherein the second mass is greater than the first mass.

Optionally, the switching device may further permit switching of the aerosol generator into an off mode, wherein in the off mode, the aerosol generator is configured not to produce aerosol during the delivery event.

Advantageously, the switching device may permit continuous variation in mass of aerosol precursor per delivery event between the normal mode and the boost mode.

Conveniently, the switching device may be configured to bias the aerosol generator from the boost mode towards the normal mode.

Optionally, the switching device may comprise a sliding switching mechanism slidable to switch the aerosol generator between the normal mode and the boost mode.

Advantageously, the aerosol generator may comprise: an aerosol generator portion configured to receive the aerosol precursor; and an air flow passage configured to direct air past the aerosol generator portion to pick up the aerosol precursor from the aerosol generator portion to form the aerosol, wherein the switching device is configured to provide relative movement between the aerosol generator portion and the airflow passage to switch the aerosol generator between the normal mode and the boost mode.

Conveniently, the switching device may be configured to provide the relative movement to vary a surface area of the aerosol generator portion located in an aerosol generation region of the airflow passage to switch the aerosol generator between the normal mode and the boost mode.

Optionally, the switching device may be configured to provide the relative movement to vary a velocity of airflow past the aerosol generator portion to switch the aerosol generator between the normal mode and the boost mode.

Advantageously, the switching device may be configured to slide the aerosol generator portion to switch the aerosol generator between the normal mode and the boost mode.

Optionally, the aerosol delivery device may comprise a storage for storing the aerosol precursor.

Conveniently, the switching device may be further configured to slide the storage with the aerosol generator portion.

Advantageously, the switching device may be configured to provide a relative bias between the aerosol generator and the airflow passage towards the normal mode.

Optionally, the aerosol generator may comprise a member, the member comprising the aerosol generator portion, wherein the member is configured to transfer the aerosol precursor to the aerosol generator portion.

Optionally, the aerosol may comprise a flavour component.

Advantageously, the aerosol may be sized to inhibit pulmonary penetration, and the aerosol is transmissible within at least one of a mammalian oral cavity and a mammalian nasal cavity.

Conveniently, the aerosol delivery device may be a consumable for a smoking substitute device.

Advantageously, the storage may comprise a reservoir, the reservoir formed of a first porous material.

Optionally, the storage may comprise a tank configured to store the aerosol precursor as a free liquid.

Advantageously, the aerosol delivery device may be a consumable for a smoking substitute device.

Conveniently, the aerosol delivery device may comprise an additional aerosol generator, the additional aerosol generator configured to produce an additional aerosol from an additional aerosol precursor during the delivery event.

Optionally, the additional aerosol generator may be configured to produce the additional aerosol comprising substantially the same mass of additional aerosol precursor per delivery event independently of the mass of aerosol precursor per delivery event.

Advantageously, the additional aerosol generator may be configured to heat the additional aerosol precursor to form the additional aerosol.

Conveniently, the second aerosol precursor may comprise an active component.

Optionally, the active component may be nicotine.

Advantageously, the additional aerosol may be sized for pulmonary penetration.

The invention includes the combination of the aspects and preferred features described except where such a combination is clearly impermissible or expressly avoided.

### Summary of the Figures

So that the invention may be understood, and so that further aspects and features thereof may be appreciated, embodiments illustrating the principles of the invention will now be discussed in further detail with reference to the accompanying figures, in which:
**Fig. 1** shows a schematic drawing of a smoking substitute system;
**Fig. 2** shows a schematic drawing of a smoking substitute system;
**Fig. 3** shows a schematic drawing of a smoking substitute system;
**Fig. 4** shows a schematic drawing of a smoking substitute system;
**Fig. 5** shows a cutaway view of a consumable;
**Fig. 6** shows a cross-sectional view of a flavour pod portion of a consumable;
**Fig. 7a** shows a partial cutaway view of a consumable with an aerosol generator portion in a normal mode;
**Fig. 7b** shows a partial cutaway view of a consumable with an aerosol generator portion in a boost mode.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

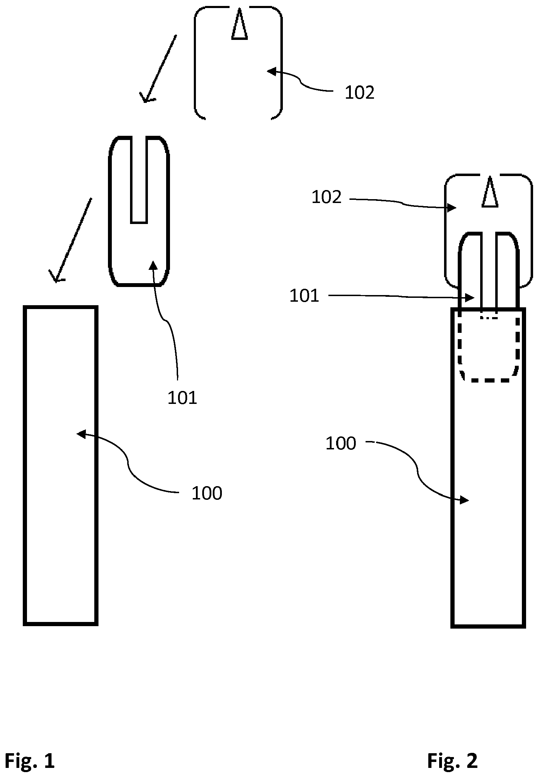

Referring to Figures 1 and 2, there is shown a smoking substitute system comprising a smoking substitute device 100. In this example, the substitute smoking system comprises a cartomiser 101 and a flavour pod 102. The cartomiser 101 may engage with the smoking substitute device 100 via a push-fit engagement, a screw-thread engagement, or a bayonet fit, for example. A cartomiser may also be referred to as a "pod". The smoking substitute system may be an aerosol delivery device according to the present invention.

The flavour pod 102 is configured to engage with the cartomiser 101 and thus with the substitute smoking device 100. The flavour pod 102 may engage with the cartomiser 101 via a push-fit engagement, a screw-thread engagement, or a bayonet fit, for example. Fig. 2 illustrates the cartomiser 101 engaged with the substitute smoking device 100, and the flavour pod 102 engaged with the cartomiser 101. As will be appreciated, in this example, the cartomiser 101 and the flavour pod 102 are distinct elements. Each of the cartomiser 101 and the flavour pod may be an aerosol delivery device according to the present invention.

As will be appreciated from the following description, the cartomiser 101 and the flavour pod 102 may alternatively be combined into a single component that implements the functionality of the cartomiser 101 and flavour pod 102. Such a single component may also be an aerosol delivery device according to the present invention. In other examples, the cartomiser may be absent, with only a flavour pod 102 present.

A "consumable" component may mean that the component is intended to be used once until exhausted, and then disposed of as waste or returned to a manufacturer for reprocessing.

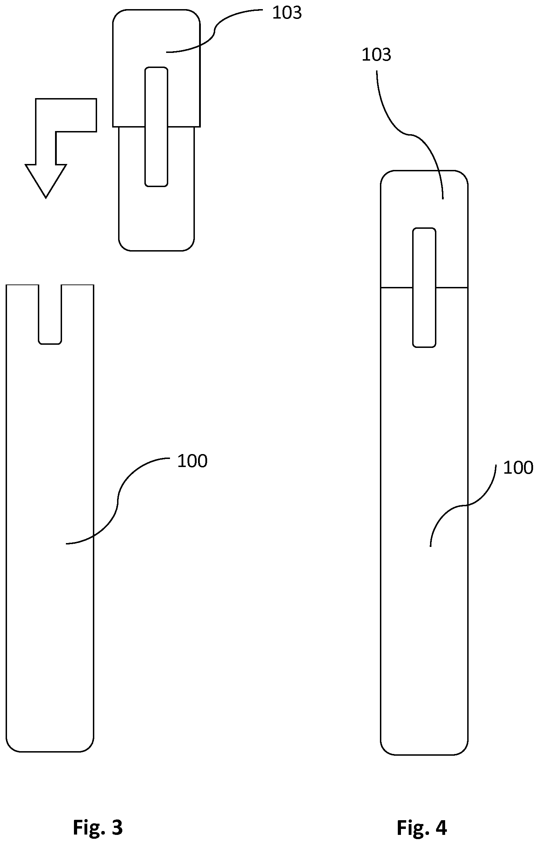

Referring to Figures 3 and 4, there is shown a smoking substitute system comprising a smoking substitute device 100 and a consumable 103. The consumable 103 combines the functionality of the cartomiser 101 and the flavour pod 102. In Figure 3, the consumable 103 and the smoking substitute device 100 are shown separated from one another. In Figure 4, the consumable 103 and the smoking substitute device 100 are engaged with each other.

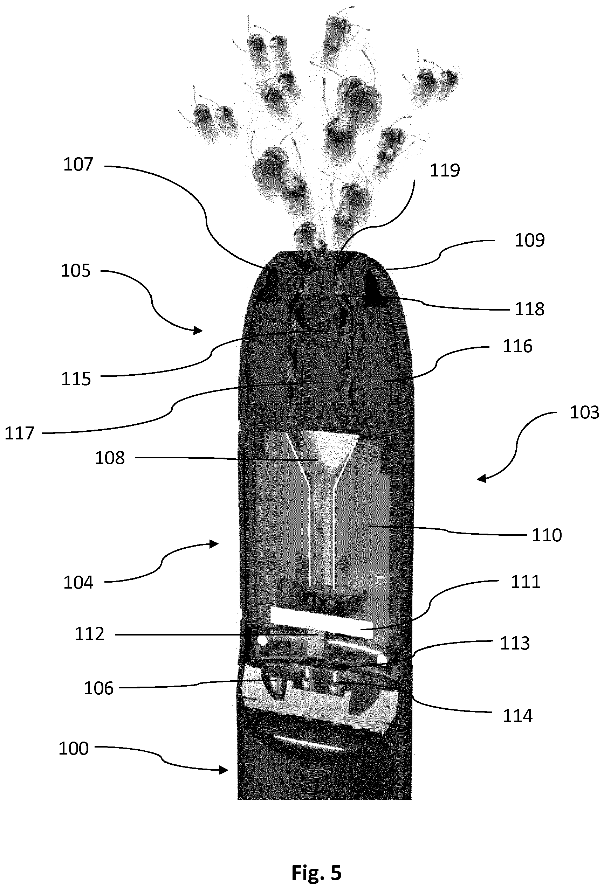

Referring to Figure 5, there is shown a consumable 103 engaged with a smoking substitute device 100 via a push-fit engagement. The consumable 103 may be considered to have two portions - a cartomiser portion 104 and a flavour pod portion 105, both of which are located within a single component (as in Figures 3 and 4).

The consumable 103 includes an upstream airflow inlet 106 and a downstream airflow outlet 107. In other examples a plurality of inlets and/or outlets are included. Between and fluidly connecting the inlet 106 and the outlet 107 there is an airflow passage 108. The outlet 107 is located at the mouthpiece 109 of the consumable 103, and is formed by a mouthpiece aperture.

As above, the consumable 103 includes a flavour pod portion 105. The flavour pod portion 105 is configured to generate a first (flavour) aerosol for output from the outlet 107 of the mouthpiece 109 of the consumable 103. The flavour pod portion 105 of the consumable 103 includes a member 115. The member 115 acts as a passive aerosol generator (i.e. an aerosol generator which does not use heat to form the aerosol, also referred to as an "aerosol generator" and a "first aerosol generator"), and is formed of a porous material. The member 115 comprises a supporting portion 117, which is located inside a housing, and an aerosol generator portion 118, which is located in the airflow passage 108. In this example, the aerosol generator portion 118 is a porous nib.

A first storage 116 (in this example a tank) for storing an aerosol precursor (i.e. "first aerosol precursor", which is a flavour liquid) is fluidly connected to the member 115. The porous nature of the member 115 means that flavour liquid from the first storage 116 is drawn into the member 115. As the first aerosol precursor in the member 115 is depleted in use, further flavour liquid is drawn from the first storage 116 into the member 115 via a wicking action.

As described above, the aerosol generator portion 118 is located within the airflow passage 108 through the consumable 103. The aerosol generator portion 118 therefore constricts or narrows the airflow passage 108. The aerosol generator portion 118 occupies some of the area of the airflow passage, resulting in constriction of the airflow passage 108. The airflow passage 108 is narrowest adjacent to the aerosol generator portion 118. Since the constriction results in increased air velocity and corresponding reduction in air pressure at the aerosol generator portion 118, the constriction is a Venturi aperture 119.

The cartomiser portion 104 of the consumable 103 includes a second storage 110 (in this example a tank) for storing a second aerosol precursor (i.e. e-liquid, which may contain nicotine). Extending into the second storage 110 is a wick 111. The wick 111 is formed from a porous wicking material (e.g. a polymer) that draws second aerosol precursor from the second storage 110 into a central region of the wick 111 that is located outside the e-liquid storage tank 110.

A heater 112 is a configured to heat the central region of the wick 111. The heater 112 includes a resistive heating filament that is coiled around the central region of the wick 111. The wick 111, the heater 112 and the e-liquid storage tank 110 together act as an active aerosol generator (i.e. an aerosol generator which uses heat to form the aerosol, also referred to as an "additional aerosol generator" and a "second aerosol generator").

As described above, the first and second aerosol generators are both at least partially located within the airflow passage 108, with the first aerosol generator downstream (with respect to air flow in use) of the second aerosol generator.

So that the consumable 103 may be supplied with electrical power for activation of the heater 112, the consumable 103 includes a pair of consumable electrical contacts 113. The consumable electrical contacts 113 are configured for electrical connection to a corresponding pair of electrical supply contacts 114 in the smoking substitute device 100. The consumable electrical contacts 113 are electrically connected to the electrical supply contacts 114 when the consumable 103 is engaged with the smoking substitute device 100. The smoking substitute device 100 includes an electrical power source (not shown), for example a battery.

In use, a user draws (or "sucks", or "pulls") on the mouthpiece 109 of the consumable 103, which causes a drop in air pressure at the outlet 107, thereby generating air flow through the inlet 106, along the airflow passage 108, out of the outlet 107 and into the user's mouth.

When the heater 112 is activated (by passing an electric current through the heating filament in response to the user drawing on the mouthpiece 109) the e-liquid located in the wick 111 adjacent to the heating filament is heated and vaporised to form a vapour. The vapour condenses to form the second aerosol within the airflow passage 108. Accordingly, the second aerosol is entrained in an airflow along the airflow flow passage 108 to the outlet 107 and ultimately out from the mouthpiece 109 for inhalation by the user when the user 10 draws on the mouthpiece 109.

The substitute smoking device 100 supplies electrical current to the consumable electrical contacts 113. This causes an electric current flow through the heating filament of the heater 112 and the heating filament heats up. As described, the heating of the heating filament causes vaporisation of the e-liquid in the wick 111 to form the second aerosol.

As the air flows up through the airflow passage 108, it encounters the aerosol generator portion 118. The constriction of the airflow passage 108 caused by the aerosol generator portion 118 results in an increase in air velocity and corresponding decrease in air pressure in the airflow in the vicinity of the porous surface 118 of the aerosol generator portion 115. The corresponding low pressure region causes the generation of the first (flavour) aerosol from the porous surface 118 of the aerosol generator portion 118. The first (flavour) aerosol is entrained into the airflow and ultimately is output from the outlet 107 of the consumable 103 and thus from the mouthpiece 109 into the user's mouth.

The first aerosol is sized to inhibit pulmonary penetration. The first aerosol is formed of particles with a mass median aerodynamic diameter that is greater than or equal to 15 microns, in particular, greater than 30 microns, more particularly greater than 50 microns, yet more particularly greater than 60 microns, and even more particularly greater than 70 microns.

The first aerosol is sized for transmission within at least one of a mammalian oral cavity and a mammalian nasal cavity. The first aerosol is formed by particles having a maximum mass median aerodynamic diameter that is less than 300 microns, in particular less than 200 microns, yet more particularly less than 100 microns. Such a range of mass median aerodynamic diameter will produce aerosols which are sufficiently small to be entrained in an airflow caused by a user drawing air through the flavour element and to enter and extend through the oral and or nasal cavity to activate the taste and/or olfactory receptors.

The second aerosol generated is sized for pulmonary penetration (i.e. to deliver an active ingredient such as nicotine to the user's lungs). The second aerosol is formed of particles having a mass median aerodynamic diameter of less than or equal to 10 microns, preferably less than 8 microns, more preferably less than 5 microns, yet more preferably less than 1 micron. Such sized aerosols tend to penetrate into a human user's pulmonary system, with smaller aerosols generally penetrating the lungs more easily. The second aerosol may also be referred to as a vapour.

The size of aerosol formed without heating is typically smaller than that formed by condensation of a vapour.

As a brief aside, it will be appreciated that the mass median aerodynamic diameter is a statistical measurement of the size of the particles/droplets in an aerosol. That is, the mass median aerodynamic diameter quantifies the size of the droplets that together form the aerosol. The mass median aerodynamic diameter may be defined as the diameter at which 50% of the particles/droplets by mass in the aerosol are larger than the mass median aerodynamic diameter and 50% of the particles/droplets by mass in the aerosol are smaller than the mass median aerodynamic diameter. The "size of the aerosol", as may be used herein, refers to the size of the particles/droplets that are comprised in the particular aerosol.

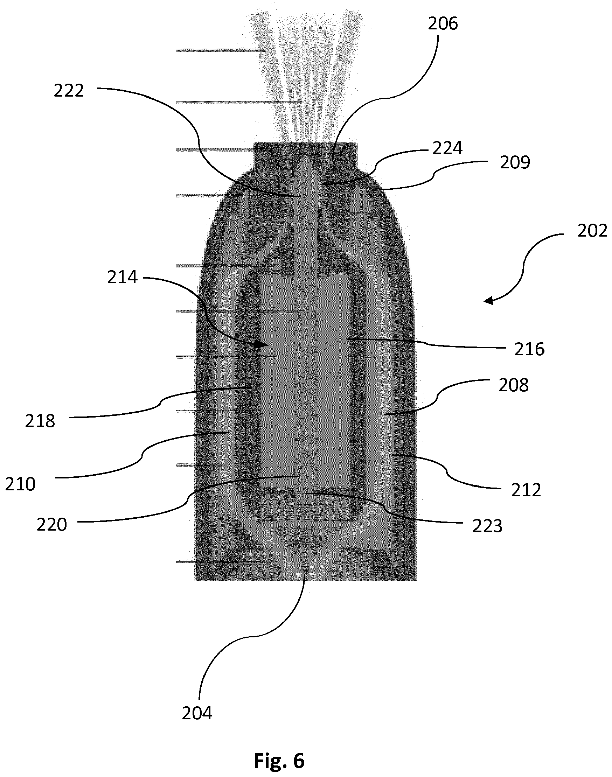

Referring to Fig. 6, there is shown a flavour pod portion 202 of a consumable, the consumable providing an aerosol delivery device in accordance with the invention. The consumable further comprises a cartomiser portion (not shown in Fig. 6) having all of the features of the cartomiser portion 104 described above with respect to Fig. 5. However, in other examples, the consumable does not comprise the cartomiser portion, and provides only flavour to the user.

The flavour pod portion 202 comprises an aerosol generator (also referred to as a "first aerosol generator"), which comprises an upstream (i.e. upstream with respect to flow of air in use) inlet 204 and a downstream (i.e. downstream with respect to flow of air in use) outlet 206. Between and fluidly connecting the inlet 204 and the outlet 206 the flavour pod portion 204 comprises an airflow passage 208. The airflow passage 208 comprises a first airflow branch 210 and a second airflow branch 212, each of the first airflow branch 210 and the second airflow branch 212 fluidly interconnecting the inlet 204 and the outlet 206. In other examples the airflow passage 208 may have an annular shape. The outlet 206 is located at the mouthpiece 209 of the consumable 103, and is also referred to as a mouthpiece aperture 206.

The flavour pod portion 202 comprises a storage 214, which stores a first aerosol precursor (also referred to as an "aerosol precursor". The storage 214 comprises a reservoir 216 located within a chamber 218. The reservoir 216 is formed of a first porous material.

The aerosol generator comprises a member 220, which comprises an aerosol generator portion 222 and a supporting portion 223. The aerosol generator portion 222 is located at a downstream end (an upper end in Fig. 6) of the member 220, while the supporting portion 223 makes up the rest of the member 220. The supporting portion 223 is elongate and substantially cylindrical. The aerosol generator portion 222 is bulb-shaped, and comprises a portion which is wider than the supporting portion 223. The aerosol generator portion 222 tapers to a tip at a downstream end of the aerosol generator portion 222.

The member 220 extends into and through the storage 214. The member 220 is in contact with the reservoir 216. More specifically, the supporting portion 223 extends into and through the storage 204 and is in contact with the reservoir 216. The member 220 is located in a substantially central position within the reservoir 216 and is substantially parallel to a central axis of the consumable. The member 220 is formed of a second porous material.

The first and second airflow branches 210, 212 are located on opposite sides of the member 220. Additionally, the first and second airflow branches 210, 212 are located on opposite sides of the reservoir 216. The first and second airflow branches 210, 212 branch in a radial outward direction (with respect to the central axis of the consumable 200) downstream of the inlet 204 to reach the opposite sides of the reservoir 216.

The aerosol generator portion 222 is located in the airflow passage 208 downstream of the first and second airflow branches 210, 212. The first and second airflow branches 210, 212 turn in a radially inward direction to merge at the member 220, at a point upstream of the aerosol generator portion 222.

The aerosol generator portion 222 is located in a narrowing section 224 of the airflow passage 208. The narrowing section 224 is downstream of the point at which the first and second airflow branches 210 212 merge, but upstream of the mouthpiece aperture 207. The mouthpiece aperture 207 flares outwardly in the downstream direction, such that a width of the mouthpiece aperture 207 increases in the downstream direction.

In use, when a user draws on the mouthpiece 209, airflow is generated through the airflow passage 208. Air (comprising the second aerosol from the cartomiser portion as explained above with respect to Fig. 5) flows through the inlet 204 before the air flow splits to flow through the first and second airflow branches 210, 212. Further downstream, the first and second airflow branches 210, 212 provide inward airflow towards the member 220 and the aerosol generator portion 222.

As air flows past the aerosol generator portion in the narrowing section 224, the velocity of the air increases, resulting in a drop in air pressure. This means that the air picks up the first aerosol precursor from the aerosol generator portion 222 to form the first aerosol. The first aerosol has the particle size and other properties described above with respect to Fig. 5.

As the first aerosol precursor is picked up by the air, the member 220 transfers further first aerosol precursor from the storage 214 to the aerosol generator portion 222. More specifically, the member 220 wicks the first aerosol precursor from the storage 214 to the aerosol generator portion 223.

In other examples, the storage 214 comprises a tank containing the first aerosol precursor as free liquid, rather than the reservoir 216 and the chamber 218. In such examples, the member 220 still extends into the tank to transfer first aerosol precursor from the tank to the aerosol generator portion 223.

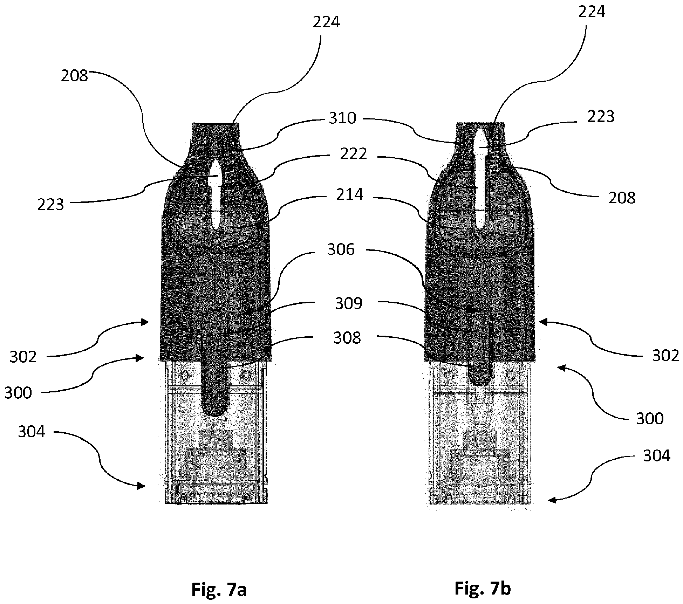

Referring to Fig. 7a and 7b, there is shown a third consumable 300. The third consumable 300 comprises a flavour pod portion 302 and a cartomiser portion 304. The third consumable 300 comprises all of the features of the second consumable (and may also comprise all of the variations of the second consumable 200 described above), and only the differences are described here. For clarity many of the reference numerals are omitted from Fig. 7a and 7b.

The flavour pod portion 302 further comprises a switching device 306. The switching device 306 comprises a button 308 and a switching mechanism (not shown). The flavour pod portion 302 comprises a recess 309 for receiving the button 308. The button 308 is located on an in use front face of the third consumable 300. The button 308 is slidable within the recess 309 upwardly and downwardly (i.e. in a downstream direction towards and an upstream direction away from the mouthpiece 209).

In other examples, the button may be slidable in a horizontal plane or may be a rotary switch. These options may help to prevent accidental removal of the third consumable 300.

The switching mechanism connects the button 308 to the storage 214 and the member 222. The switching device 306 further comprises a bias device, which is a spring 310. The spring 310 is connected to the storage 214 and an underside of the mouthpiece 209. The spring 310 is configured to push the storage 214 (and hence the member 222) away from the mouthpiece 209.

The third consumable 300 comprises an aerosol generator, which comprises all of the features of the first aerosol generator described above. Fig. 7a shows the aerosol generator portion 223 of the aerosol generator in a normal mode, while Fig. 7b shows the aerosol generator portion 223 in a boost mode.

In the boost mode, a larger area of the aerosol generator portion 223 is located in an aerosol generation region of the air flow passage 208 than is the case in the normal mode. The aerosol generation region of the air flow passage 208 may be defined as a region in which the velocity of air flow is sufficiently high to generate the first aerosol in use. In the present example, the aerosol generation region of the air flow passage 208 is the narrowing section 224.

Additionally, in the boost mode, the aerosol generator portion 223 provides an increased area of constriction in the narrowing section 224 than is the case in the normal mode, which increases the velocity of airflow past the aerosol generator portion 223 in use compared to the normal mode.

Each of increasing the surface area of the aerosol generator portion 223 in the aerosol generation region and increasing the velocity of airflow past the aerosol generator portion 223 result in increased mass of first aerosol precursor in the first aerosol.

The button 308 therefore allows the user to provide relative movement between the aerosol generator portion 223 and the airflow passage 208. This allows the user to vary/adjust a mass flow of aerosol precursor produced per delivery event (i.e. per puff taken by the user).

In the present example, the normal mode is the position in which the surface area of aerosol generator portion 223 located in the aerosol generation region is at a minimum. Additionally, the normal mode is the position in which the constriction in the narrowing section 224 is at a minimum.

In the present example, the boost mode is the position in which the surface area of aerosol generator portion 223 located in the aerosol generation region is at a maximum. Additionally, the boost mode is the position in which the constriction in the narrowing section 224 is at a maximum.

In use, to switch the aerosol generator portion 223 from the normal mode to the boost mode, the user slides the button 308 in an upward direction (i.e. in a downstream direction and towards the mouthpiece). This effects relative movement between the aerosol generator portion 223 and the airflow passage 208. More specifically, this effects sliding of the storage 214 and the member 222. As the button slides in the upward direction, the aerosol generator portion 223 slides further into the narrowing section 224 until the boost mode position is reached.

The user may slide the button 308 to any extent between the normal mode and the boost mode, permitting continuous variation in position of the aerosol generation portion 223 and continuous variation in mass of first aerosol precursor per delivery event between the normal mode and the boost mode. In general, as the user pushes the button 308 upwards, the surface area of the aerosol generator portion 223 in the aerosol generation region (in this case the narrowing section 224) increases. Additionally, as the user pushes the button 308 upwards, the aerosol generator portion 223 increases the area of constriction in the narrowing section 224, thereby increasing the velocity of airflow past the aerosol generator portion 223.

When the button 308 is released by the user, the spring 310 forces the aerosol generator portion 223 back into the normal mode.

In each of the normal mode and the boost mode (and any position of the aerosol generation portion 223 between the normal mode and the boost mode), the aerosol generator generally operates in the same way as the aerosol generator described above with respect to Fig. 6, with air flowing past the aerosol generator portion 223 to pick up aerosol precursor to generate aerosol during a delivery event when a user inhales.

In the present example, in the normal mode, the aerosol generator produces an aerosol comprises a first mass of aerosol precursor per delivery event. The first mass is less than 10 mg. More specifically, the first mass is less than 8 mg. More specifically, the first mass is less than 6 mg.

The first mass is at least 2 mg. More specifically, the first mass is at least 3 mg. More specifically, the first mass is at least 4 mg. More specifically, the first mass is substantially 5 mg.

In the boost mode, the aerosol generator produces an aerosol which comprises a second mass of aerosol precursor per delivery event. The second mass is greater than the first mass. The second mass is at least 10 mg. More specifically, the second mass is at least 12 mg. More specifically, the second mass is at least 14 mg.

The second mass is not more than 20 mg. More specifically, the second mass is not more than 18 mg. More specifically, the second mass is not more than 16 mg. More specifically, the second mass is substantially 15 mg.

The second aerosol generator (or "additional aerosol generator") of the cartomiser portion continues to produce second aerosol (or "additional aerosol") comprising substantially the same mass of second aerosol precursor (or "additional aerosol precursor") per delivery event regardless of the mode of the aerosol generator and the position of the aerosol generator portion 223.

In other examples, the switching device permits the aerosol generator portion 223 to be moved beyond the normal mode, to a position in which in the aerosol generator portion 223 has substantially zero surface area in the generation region. In this position, substantially zero aerosol precursor is produced during the delivery event, thereby providing an off mode.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the words "have", "comprise", and "include", and variations such as "having", "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means, for example, +/- 10%.

The words "preferred" and "preferably" are used herein refer to embodiments of the invention that may provide certain benefits under some circumstances. It is to be appreciated, however, that other embodiments may also be preferred under the same or different circumstances. The recitation of one or more preferred embodiments therefore does not mean or imply that other embodiments are not useful, and is not intended to exclude other embodiments from the scope of the disclosure, or from the scope of the claims.

## Claims

1. An aerosol delivery device comprising:
an aerosol generator configured to produce an aerosol from an aerosol precursor during a delivery event, wherein the aerosol comprises a flavour component; and
a switching device permitting switching of the aerosol generator between a normal mode and a boost mode, wherein the aerosol generator is configured to:
in the normal mode, produce aerosol comprising a first mass of aerosol precursor per delivery event; and
in the boost mode, produce aerosol comprising a second mass of aerosol precursor per delivery event,
wherein the second mass is greater than the first mass.

2. An aerosol delivery device according to claim 1, wherein the switching device further permits switching of the aerosol generator into an off mode, wherein in the off mode, the aerosol generator is configured not to produce aerosol during the delivery event.

3. An aerosol delivery device according to claim 1 or claim 2, wherein the switching device permits continuous variation in mass of aerosol precursor per delivery event between the normal mode and the boost mode.

4. An aerosol delivery device according to any one of the preceding claims, wherein the switching device is configured to bias the aerosol generator from the boost mode towards the normal mode.

5. An aerosol delivery device according to any one of the preceding claims, wherein the switching device comprises a sliding switching mechanism slidable to switch the aerosol generator between the normal mode and the boost mode.

6. An aerosol delivery device according to any one of the preceding claims, wherein the aerosol generator comprises:
an aerosol generator portion configured to receive the aerosol precursor; and
an air flow passage configured to direct air past the aerosol generator portion to pick up the aerosol precursor from the aerosol generator portion to form the aerosol,
wherein the switching device is configured to provide relative movement between the aerosol generator portion and the airflow passage to switch the aerosol generator between the normal mode and the boost mode.

7. An aerosol delivery device according to claim 6, wherein the switching device is configured to provide the relative movement to vary a surface area of the aerosol generator portion located in an aerosol generation region of the airflow passage to switch the aerosol generator between the normal mode and the boost mode.

8. An aerosol delivery device according to claim 6 or claim 7, wherein the switching device is configured to provide the relative movement to vary a velocity of airflow past the aerosol generator portion to switch the aerosol generator between the normal mode and the boost mode.

9. An aerosol delivery device according to any one claims 6 to 8, wherein the switching device is configured to slide the aerosol generator portion to switch the aerosol generator between the normal mode and the boost mode.

10. An aerosol delivery device according to claim 9, wherein the aerosol delivery device comprises a storage for storing the aerosol precursor, wherein the switching device is further configured to slide the storage with the aerosol generator portion.

11. An aerosol delivery device according to claim any one of the preceding claims, wherein the aerosol generator comprises a member, the member comprising the aerosol generator portion, wherein the member is configured to transfer the aerosol precursor to the aerosol generator portion.

12. An aerosol delivery device according to any preceding claim, wherein the aerosol delivery device is a consumable for a smoking substitute device.

13. An aerosol delivery device according to any preceding claim and further comprising an additional aerosol generator, the additional aerosol generator configured to produce an additional aerosol from an additional aerosol precursor during the delivery event.

14. An aerosol delivery device according to claim 13, wherein the additional aerosol generator is configured to produce the additional aerosol comprising substantially the same mass of additional aerosol precursor per delivery event in the normal mode and in the boost mode.

15. An aerosol delivery device according to claim 14, wherein the additional aerosol generator is configured to heat the additional aerosol precursor to form the additional aerosol.
